# EUROPEAN PATENT SPECIFICATION

(11) **EP 2 435 126 B1**
(45) Date of publication and mention of the grant of the patent: **13.03.2019**
(21) Application number: 10781062.4
(22) Date of filing: 24.05.2010
(51) Int. Cl.: A61M 35/00, A45D 34/04

(54) **APPLICATOR TIP ASSEMBLY FOR SKIN FORMULATION APPLICATOR**
APPLIKATORSPITZENANORDNUNG FÜR EINEN HAUTREZEPTURAPPLIKATOR
ENSEMBLE EMBOUT APPLICATEUR POUR APPLICATEUR DE FORMULATIONS DESTINÉES À LA PEAU

(30) Priority: 29.05.2009 US 474426
(43) Date of publication of application: 04.04.2012
(73) Proprietor: L'Oréal SA, 75008 Paris (FR)
(72) Inventor: REISHUS, Richard A., Renton, WA 98058 (US); ITANO, Michael M., Seattle, WA 98106 (US); PILCHER, Kenneth A., Seattle, WA 98107 (US)
(74) Representative: Faulkner, Thomas John
(86) International application number: PCT/US2010/035968
(87) International publication number: WO 2010/138457

(56) References cited:
- WO-A1-2009/152056
- FR-A1- 2 918 545
- US-A- 3 749 438
- US-A- 4 225 254
- US-A- 4 878 900
- US-A- 5 322 518
- US-A1- 2004 015 139
- US-A1- 2004 015 139
- US-A1- 2008 125 682
- US-A1- 2009 124 985
- US-B2- 7 055 807

## Description

### TECHNICAL FIELD

This invention relates generally to appliances for applying skin treatment formulations to the facial skin area of the body, and more particularly concerns a replaceable applicator tip assembly which fits on to a drive end of the appliance motor.

### BACKGROUND OF THE INVENTION

In an electric power appliance capable of applying various skin formulations, including skin treatment formulations, to a selected skin area, such as the facial skin area of the body, the applicator tip which actually contacts the skin with the formulation has a relatively short life and hence requires replacement from time to time. Such an applicator tip must have a very soft forward portion to maintain comfortable contact with the user's skin when applying the formulation. A user who experiences discomfort will be unlikely to continue to use the appliance. The applicator tip must also be readily securable to and removable from a drive end of the appliance motor. The replaceable applicator tip must reliably couple with the motor drive end so as to withstand an oscillating action of the motor drive without resulting in substantial noise or lost motion. The applicator assembly shown and described herein accomplishes such desired objectives.
The document US 2004/0015139 describes an apparatus for treating skin using abrasion lotion with particulate. The apparatus includes a skin contact surface having raised bosses that may have an arcuate profile. A motor is provided for rotating the skin contact surface so that the contact surface impresses and urges particulate across the skin.

### SUMMARY OF THE INVENTION

Accordingly, disclosed herein is an applicator tip assembly according to claim 1.

### BRIEF DESCRIPTION OF THE DRAWINGS

FIG. 1 is a schematic view of a skin formulation applicator.
FIGS. 2, 3 and 4 are elevational, cross-sectional and bottom views, respectively, of one embodiment of a new applicator tip assembly for use on a skin formulation applicator.
FIGS. 5, 6, 7 and 8 are elevational, perspective and two bottom views, respectively, of a second embodiment of the applicator tip assembly.
FIGS. 8, 9, 10 and 11 are elevational, perspective and cross-sectional views, respectively, of a third embodiment of the tip applicator assembly.

### BEST MODE FOR CARRYING OUT THE INVENTION

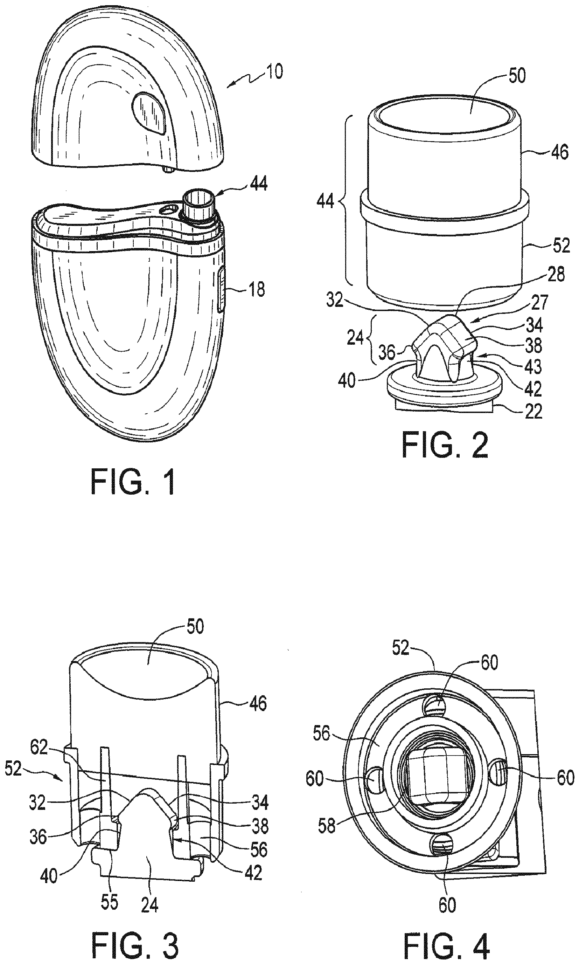

FIG. 1 shows in general an applicator appliance 10 for applying skin formulations to various areas of the skin, in particular the facial area. The applicator 10 operates in a sonic frequency range of 50-200 Hz with a preferred range of 100-140 Hz. The applicator 10, referred to as a sonic skin applicator, includes a motor, a battery and a microprocessor controller, which are not shown specifically in FIG. 1. Reference is hereby made to copending patent application Ser. No. 12/135,887. Applicator 10 is controlled by an on/off switch 18. Extending from the motor is a motor drive end member.

Removably mounted on the motor drive end member is an applicator tip assembly shown generally at 44 in FIG. 1, which comprises the subject matter of the present application. The applicator tip assembly has a limited life, and hence must be replaced at regular intervals during the lifetime of the applicator appliance. The drive end member of the motor oscillates in operation back and forth through a selected distance, typically in the range of 0.025-0.19 cm (0.010-0.075 inches), with a preferred range of 0.05-0.09 cm (0.020-0.035 inches). The applicator tip assembly 44, being firmly coupled to the drive end member, thus also oscillates similarly in operation. The mechanical properties of the tip applicator assembly, including the
arrangement for removably attaching the applicator tip assembly to the drive end member are important to the overall function of the applicator appliance.

In general, the applicator tip assembly, shown herein in several embodiments, includes a tip portion which comes into direct contract with the skin of the user when applying the skin formulations, and must be very soft in order to preserve comfortable contact between the tip portion and the user. The applicator tip assembly also includes a base portion which must be rigid enough to attach firmly to the drive end member of the motor; once attached, the applicator tip assembly must remain sufficiently tightly coupled to the drive end member of the motor to withstand the oscillating action of the resonating motor, without lost motion or creating significant noise, resulting in quiet, reliable operation. Further, the applicator tip assembly as a unit must be easy to install onto and remove from the drive end member without the use of special tools or complicated maneuvering of the assembly. The applicator tip assembly is thus conveniently replaceable. Installation and removal of successive applicator tip assemblies must not damage the drive end element of the motor. In all the embodiments herein, the applicator tip assembly is designed to flex when installed or removed from the drive end member of the motor. This insures that the more rigid appliance motor is not damaged by repeated tip assembly installations and removals. Still further, the soft tip portion is arranged and secured to the base portion so that the two will not separate during operation of the appliance.

Referring now in the embodiment of Figures 2-4, drive end member 22 extending from the appliance motor has a square or rectangular cross-section and has the form of a double-tapered protrusion 24. While in general a square cross-section is preferred, it could also be rectangular. A first taper 27 is at the very forward end of protrusion 24. The taper 27 is rounded at its most forward point 28 and then tapers away at an angle between tapered surfaces 32 and 34 in the range of 70-110°, with a nominal angle of 90°. The tapered surfaces 32 and 34 are generally flat, although they do round inwardly at the lower ends thereof, defining rounded edges 36 and 38.

Tapering slightly outwardly from the inner ends of rounded edges 36 and 38 are tapered surfaces 40 and 42, defining a second taper 43. The combination of the two tapers 27 and 43 permits a secure attachment between the applicator tip assembly and the drive end member of the motor, while at the same time permitting the applicator tip assembly to be conveniently installed and then removed, as described more specifically below.

FIGS. 2, 3 and 4 show an applicator tip assembly 44 with a soft tip portion 46 which, in the embodiment shown, is circular in cross-section, approximately 0.10 cm (0.40 inches) in diameter, and is approximately 0.5 cm (0.20 inches) high at the peripheral edge thereof. These dimensions can vary, depending upon the particular application. The tip portion 46 includes a concave forward surface 50 which is designed to hold skin formulations. As indicated above, it is important that the tip portion be soft for comfort to the user and to improve the application and effective use of the formulation. Preferably, the tip portion material is a very low durometer silicone, elastomeric or similar rubber material, which can be either injection molded or cast onto the base portion of the tip assembly. In the embodiment shown, the material is a Shore scale OO Durometer 30 prosthetic grade silicone. This material has a very positive feel as well as good function. The good function is due to the fact that the flexibility of the material is similar to that of human skin and thus transmits motion and force efficiently.

The other portion of the tip assembly is a base portion shown generally at 52. In this embodiment, base portion 52 material is flexible, but not particularly soft. A high or low density polyethylene is suitable, as well as other comparable materials. The base portion 52 includes a lower element 56 having a central opening 58. The lower element also includes four equally spaced holes 60-60 outboard of opening 58, the purpose of which is to allow trapped air to escape during the casting process and allow the casting material to fill and adhere to the interior vertical walls of base portion 52.

The lower member 56 is flexible enough to permit the base portion 52 of the applicator tip assembly to be readily inserted onto protrusion 24. Opening 58 is somewhat smaller than the largest diameter dimension of rounded edges 36 and 38, so that when the applicator tip assembly is pushed past the first taper 27 to the point where the lower surface of the base portion abuts surface 55 of the drive end of the motor (FIG. 3), the lower element 56 of the base portion 52 rebounds such that the edge of opening 58 engages and is retained by an upper portion of the second taper 43. The material comprising the lower element 56 of base portion 52 is flexible enough that it deforms under pressure so as to permit the first taper 27 of protrusion 24 to move through the opening, permitting or allowing convenient installation and removal of the applicator tip assembly. When the lower element 56 rebounds to its original configuration following proper installation of the assembly, the applicator tip assembly is firmly latched onto the drive end member of the motor.

Extending upwardly from the upper surface of the lower element 56, outboard of opening 58, is a ring or cylinder 62, to which the soft tip portion 46 is either molded or cast, which results in a secure connection between the two portions of the assembly, *i.e.* so that the tip portion and the base assembly are securely joined and hence do not separate during normal oscillating action of the drive end member of the motor.

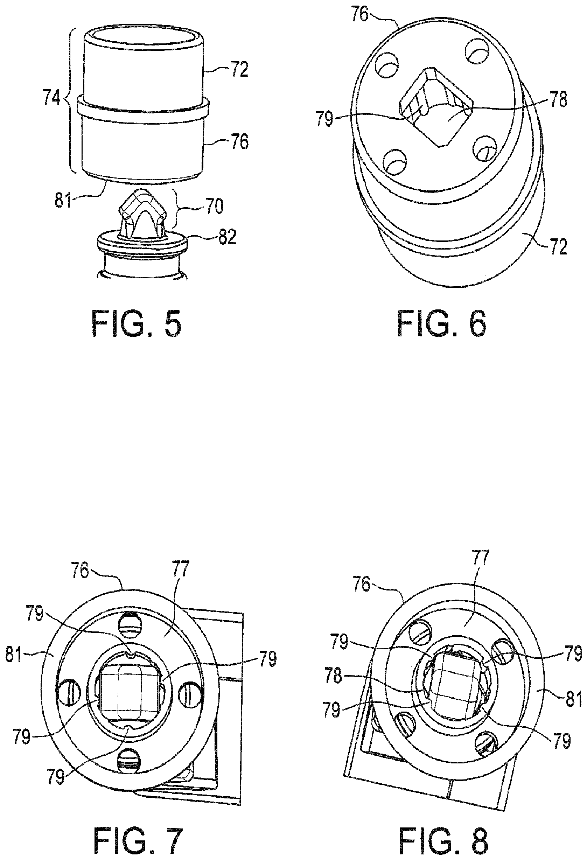

A second embodiment, shown in Figures 5-8, uses a similarly configured protrusion at the end of the drive end member to that of the embodiment of Figures 2-4. A square or a rectangular double-tapered protrusion is shown at 70, identical to that of Figure 2. In the second embodiment, tip portion 72 of the applicator tip assembly 74 is basically the same as tip portion 46 in the embodiment of Figures 2-4. Base portion 76 comprises a less flexible material than the base portion of the embodiment of Figures 2-4. ABS or similar materials are used in this embodiment. Lower element 77 of base portion 76 includes an opening 78 into which protrusion 70 freely fits. A square opening is generally preferred, although a rectangular shape can be used as well. In this embodiment, a number of small ribs 79 are molded onto an interior ring or cylindrical member 80 which extends upwardly from the lower element 76. The upper part of ring member 80 receives the tip portion, which is molded or cast thereon.

In this embodiment, base portion 76 is inserted onto protrusion 70 until lower ring surface 81 of the base portion abuts surface 82 of the motor drive end member. In this position, the side edges of the first tapered portion are within the ribbed ring member 80 then rotated through an angle in the range of 30-60°, with a preferred angle of 45°, which latches the applicator tip assembly onto the protrusion by an interference fit between ribs 79 and the edges of the first taper of protrusion 70. When the applicator tip assembly is to be removed for replacement, the tip assembly is rotated in the opposite direction the same amount and then pulled away from the protrusion.

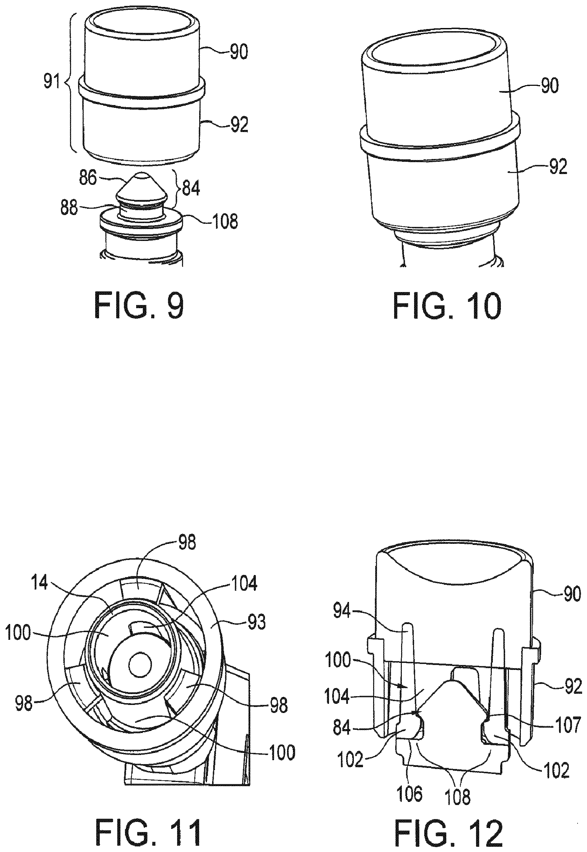

Another embodiment is shown in Figures 9-12. In this embodiment, protrusion 84 at the drive end member has a different configuration. A forward section/taper 86 of the protrusion is conical, while a rear section 88 is generally cylindrical in cross-section. In this arrangement, tip portion 90 of the applicator assembly 91 has the same characteristics set forth for the above embodiments. Base portion 92 comprises a relatively rigid material such as ABS or similar material. In this embodiment, base portion 92 includes an outer cylindrical wall 93 and an inner cylindrical ring 94. Extending from inner ring 94 to outer cylindrical wall 93 are three equally spaced web elements 98-98, located near the upper edge of the outer wall 93. This provides structural stability between inner ring 94 and outer wall 93. Extending downwardly from inner ring member 94, between the web elements 98, are three spaced vertical legs 100-100, typically equally spaced. The lower edges of the vertical legs are free to move outwardly under pressure, as the lower edges are not secured to wall 93. They are resilient, however, so that when outward pressure on the vertical legs is released they snap back into their initial, substantially vertical position. While three web elements and three vertical legs are disclosed, it should be understood that other numbers of those elements could be used, for example 2 or 4 elements.

The lower edge portions 102 of the three vertical legs are rounded and extend slightly into the cylindrical opening 104 defined partially by the vertical legs 100, as well as slightly outboard of the remainder of the vertical legs, as shown most clearly in Figure 11. The vertical legs 100 are configured relative to protrusion 84 such that the base portion of the applicator tip assembly can be snapped onto the protrusion, with a portion of the lower edges 102 of the vertical legs abutting lower edge 107 of taper 86 of the protrusion as well as horizontal surface 108 of the drive end member, tending to hold the applicator tip assembly firmly into place on the protrusion.

The tip portion 90 is molded or cast onto the upper part of inner ring 94 of base portion 92, securing the two parts together. When the applicator tip assembly is to be removed, longitudinal force is exerted outwardly on the applicator tip assembly, which results in the vertical legs 100 being temporarily spread outwardly, permitting the applicator tip assembly to be removed from protrusion 84.

Hence, a replaceable applicator tip assembly for use with an applicator for skin formulations has been disclosed. It includes a tip portion which is securely molded or cast onto a base portion, with the tip portion being quite soft so as to provide comfort and efficacy when in contact with the user's skin. The base portion of the applicator tip assembly is relatively rigid, permitting a secure, dynamic connection with the drive end element from the applicator motor, while at the same time permitting convenient installation and removal of the tip assembly onto the protrusion. As indicated above the tip applicator assembly is conveniently replaceable, since the life of the applicator is considerably longer than the life of the tip assembly. The tip assembly is easy to install and remove, without the need for tools, yet is secure when installed, without loss of motion and providing quiet operation. Several embodiments have been disclosed for accomplishing this combination of desired functions.

Although a preferred embodiment has been disclosed for purposes of illustration, it should be understood that various changes and modifications and substitutions could be made in the preferred embodiment without departing from the scope of the claims which follow:

## Claims

1. An applicator tip assembly (44, 74, 91) for use in an appliance (10) for applying skin formulations, comprising:
a base portion (52, 76, 92), sufficiently rigid to be insertable onto and removable from a drive end member (22) of a motor for the appliance and to move with the action of the motor drive end member (22); and
a tip portion (46, 72, 90), wherein the base portion and the tip portion are securely mated so that they move together by action of the motor,
**characterised in that** the tip portion has a flexibility similar to that of human skin and is sufficiently soft to provide comfortable contact with the facial skin of a user, and wherein the action of the motor drive end member is oscillating in a linear direction, toward and away from the skin of a user, and wherein the tip portion includes a single concave forward surface (50) for receiving skin formulations.

2. The applicator tip assembly of claim 1, wherein the tip potion is soft silicone or rubber, approximately Shore scale OO Durometer 30.

3. The applicator tip assembly of claim 1 or 2, wherein the oscillation is within the frequency range of 50-200 Hz.

4. The applicator tip assembly of claim 3, wherein the oscillation is within the range of 100-140 Hz.

5. The applicator tip assembly of any of the preceding claims, wherein the tip portion is molded or cast onto a tip attachment element or elements which extend upwardly from the base portion.

6. An appliance (10) for applying skin formulations comprising the applicator tip assembly of any of the preceding claims and a motor having a drive end member (22) configured for connection with the base portion, wherein the drive end member (22) includes a protrusion (24, 70, 84) at a forward end thereof.

7. The appliance of claim 6, wherein the protrusion is square or rectangular in cross-section and has a forward larger portion and a rear smaller portion.

8. The appliance of claim 7, wherein the larger portion and the smaller portions both are tapered.

9. The appliance of claim 6, wherein the protrusion has a conical forward section and a smaller rear section.

10. The appliance of claim 6, wherein the base portion (52) includes a lower element (56), having an opening (58) which is configured and arranged to be smaller than the protrusion but sufficiently flexible to permit installation thereof onto the protrusion, wherein an inner edge of the opening abuts a rear taper portion of the protrusion, securely holding the applicator tip assembly onto the protrusion.

11. The applicator tip assembly of claim 5, wherein the attachment element is a continuous ring (62).

12. The applicator tip assembly of claim 1, wherein the base portion (76) includes a lower element (77) with a central opening (78) through which the protrusion can move, and an inner surface having rib portions (79) which are arranged and configured to latch and securely hold the tip assembly onto the protrusion following insertion of the tip assembly and rotation thereof a partial turn.

13. The appliance of claim 6, wherein the base portion (92) includes an outer wall (93) and an inner ring (94) secured to the outer wall by spaced web members (98), the inner ring including flexible legs/elements (100) between the web members which extend downwardly and are arranged and configured to permit a first taper portion of the protrusion to move therethrough by spreading the leg elements outwardly, wherein the leg elements spring back against the protrusion, holding the tip assembly securely onto the protrusion.

14. The appliance of claim 13, wherein the leg elements are equally spaced and sufficiently flexible to permit the tip assembly to be removed from and installed onto the protrusion, while holding the tip assembly against the protrusion in operation to prevent lost motion.

## Patentansprüche

1. Applikatorspitzenanordnung (44, 74, 91) zur Verwendung in einer Vorrichtung (10) zum Anwenden von Hautrezepturen, umfassend:
einen Basisabschnitt (52, 76, 92), welcher hinreichend starr ist, um einsetzbar in ein und entfernbar von einem Antriebsendelement (22) eines Motors für die Vorrichtung (10) zu sein und mit der Bewegung des Motor-Antriebsendelements (22) bewegt zu werden; und
einen Spitzenabschnitt (46, 72, 90), wobei der Basisabschnitt und der Spitzenabschnitt fest verbunden sind, so dass sie zusammen durch die Bewegung des Motors bewegt werden,
**dadurch gekennzeichnet, dass** der Spitzenabschnitt eine Flexibilität vergleichbar zur menschlichen Haut hat und hinreichend weich ist, um einen komfortablen Kontakt mit der Gesichtshaut eines Benutzers zu ermöglichen, und wobei die Bewegung des Motor-Antriebsendelements oszillierend in einer linearen Richtung in Richtung auf die und weg von der Haut des Benutzers ist, und wobei der Spitzenabschnitt eine einzige konkave Oberfläche (50) zur Aufnahme der Hautrezeptur umfasst.

2. Applikatorspitzenanordnung nach Anspruch 1, wobei der Spitzenabschnitt weiches Silikon oder Gummi mit einem Wert von etwa 30 auf der Shore-Durometerskala OO ist.

3. Applikatorspitzenanordnung nach Anspruch 1 oder 2, wobei die Oszillation eine Frequenz im Bereich von 50 bis 200 Hz hat.

4. Applikatorspitzenanordnung nach Anspruch 3, wobei die Oszillation eine Frequenz im Bereich von 100 bis 140 Hz hat.

5. Applikatorspitzenanordnung nach einem der vorstehenden Ansprüche, wobei der Spitzenabschnitt geformt oder gegossen ist auf ein oder mehrere Spitzenbefestigungselemente, welche sich vom Basisabschnitt nach oben erstrecken.

6. Vorrichtung (10) zum Anwenden von Hautrezepturen, umfassend die Applikatorspitzenanordnung nach einem der vorstehenden Ansprüche und einen Motor mit einem Antriebsendelement (22) eingerichtet zum Verbinden mit dem Basisabschnitt, wobei das Antriebsendelement (22) an einem vorderen Ende davon einen Vorsprung (24, 70, 84) umfasst.

7. Vorrichtung nach Anspruch 6, wobei der Vorsprung im Querschnitt quadratisch oder rechteckig ist und einen größeren Vorderabschnitt und einen kleineren Hinterabschnitt aufweist.

8. Vorrichtung nach Anspruch 7, wobei sowohl der Vorderabschnitt als auch der Hinterabschnitt konisch sind.

9. Vorrichtung nach Anspruch 6, wobei der Vorsprung einen kegelförmigen Vorderbereich und einen kleineren Hinterbereich aufweist.

10. Vorrichtung nach Anspruch 6, wobei der Basisabschnitt (52) ein unteres Element (56) umfasst, welches eine Öffnung (58) hat, die eingerichtet und angeordnet ist um kleiner zu sein als der Vorsprung aber hinreichend flexibel, um auf dem Vorsprung angebracht zu werden, wobei eine innere Kante der Öffnung an einen hinteren konischen Abschnitt des Vorsprungs anstößt, wodurch die Applikatorspitzenanordnung fest an dem Vorsprung gehalten wird.

11. Applikatorspitzenanordnung nach Anspruch 5, wobei das Befestigungselement ein durchgehender Ring (62) ist.

12. Applikatorspitzenanordnung nach Anspruch 1, wobei der Basisabschnitt (76) ein unteres Element (77) mit einer zentralen Öffnung (78), durch welche der Vorsprung bewegt werden kann, und eine innere Oberfläche (79) mit Rippenabschnitten (79), welche angeordnet und eingerichtet sind den Spitzenabschnitt nach Einsetzen des Spitzenabschnitts und Rotation desselben mit einer teilweisen Umdrehung zu sperren und fest auf dem Vorsprung zu halten, umfasst.

13. Vorrichtung nach Anspruch 6, wobei der Basisabschnitt (92) eine äußere Wand (93) und einen inneren Ring (94), welcher durch beabstandete Netzelemente (98) an der äußeren Wand befestigt ist, umfasst, wobei der innere Ring flexible Beine/Elemente (100) zwischen den Netzelementen umfasst, welche sich nach unten erstrecken und angeordnet und eingerichtet sind, um einem ersten konischen Abschnitt des Vorsprungs zu ermöglichen, sich durch diese zu bewegen, indem die Beinelemente nach außen gespreizt werden, wobei die Beinelemente gegen den Vorsprung zurückspringen und die Spitzenanordnung fest an dem Vorsprung zu halten.

14. Vorrichtung nach Anspruch 13, wobei die Beinelemente gleichmäßig beabstandet und hinreichend flexibel sind, um der Spitzenanordnung zu ermöglichen von dem Vorsprung entfernt zu werden von und an dem Vorsprung angebracht zu werden, während die Spitzenanordnung gegen den Vorsprung gehalten wird, um eine Verlierbewegung zu vermeiden.

## Revendications

1. Ensemble formant embout applicateur (44, 74, 91) destiné à être utilisé dans un appareil (10) pour appliquer des formulations pour la peau, comprenant :
une partie de base (52, 76, 92), suffisamment rigide pour pouvoir être insérée sur un élément d'extrémité d'entraînement (22) d'un moteur pour l'appareil, et pour pouvoir en être retirée, et pour se déplacer sous l'action de l'élément d'extrémité d'entraînement de moteur (22) ; et
une partie d'embout (46, 72, 90), dans lequel la partie de base et la partie d'embout sont appariées de manière sûre de sorte qu'elles se déplacent ensemble par l'action du moteur,
**caractérisé en ce que** la partie d'embout a une souplesse similaire à celle de la peau humaine et est suffisamment douce pour permettre un contact confortable avec la peau du visage d'un utilisateur, et dans lequel l'action de l'élément d'extrémité d'entraînement de moteur oscille dans une direction linéaire, vers et loin de la peau d'un utilisateur, et dans lequel la partie d'embout comprend une surface avant concave unique (50) destinée à recevoir des formulations pour la peau.

2. Ensemble formant embout applicateur selon la revendication 1, dans lequel la partie d'embout est en silicone souple ou en caoutchouc, approximativement d'une dureté de 30 au duromètre sur l'échelle Shore 00.

3. Ensemble formant embout applicateur selon la revendication 1 ou 2, dans lequel l'oscillation est dans la plage de fréquences de 50 à 200 Hz.

4. Ensemble formant embout applicateur selon la revendication 3, dans lequel l'oscillation est dans la plage de 100 à 140 Hz.

5. Ensemble formant embout applicateur selon l'une quelconque des revendications précédentes, dans lequel la partie d'embout est moulée ou coulée sur un ou plusieurs éléments de fixation d'embout qui s'étendent vers le haut depuis la partie de base.

6. Appareil (10) pour appliquer des formulations pour la peau comprenant l'ensemble formant embout applicateur selon l'une quelconque des revendications précédentes et un moteur ayant un élément d'extrémité d'entraînement (22) configuré pour une connexion à la partie de base, dans lequel l'élément d'extrémité d'entraînement (22) comprend une saillie (24, 70, 84) à une extrémité avant de celui-ci.

7. Appareil selon la revendication 6, dans lequel la saillie a une section transversale carrée ou rectangulaire et a une partie avant plus grande et une partie arrière plus petite.

8. Appareil selon la revendication 7, dans lequel la partie plus grande et les parties plus petites sont effilées.

9. Appareil selon la revendication 6, dans lequel la saillie a une section avant conique et une section arrière plus petite.

10. Appareil selon la revendication 6, dans lequel la partie de base (52) comprend un élément inférieur (56) ayant une ouverture (58) qui est configurée et agencée pour être plus petite que la saillie mais suffisamment souple pour permettre son installation sur la saillie, dans lequel un bord intérieur de l'ouverture vient en butée contre une partie effilée arrière de la saillie, en retenant de manière sécurisée l'ensemble formant embout applicateur sur la saillie.

11. Ensemble formant embout applicateur selon la revendication 5, dans lequel l'élément de fixation est un anneau continu (62).

12. Ensemble formant embout applicateur selon la revendication 1, dans lequel la partie de base (76) comprend un élément inférieur (77) avec une ouverture centrale (78) à travers laquelle la saillie peut se déplacer, et une surface intérieure ayant des parties de nervure (79) qui sont agencées et configurées pour verrouiller et maintenir fermement l'ensemble formant embout sur la saillie après insertion de l'ensemble formant embout et rotation de celui-ci d'un tour partiel.

13. Appareil selon la revendication 6, dans lequel la partie de base (92) comprend une paroi extérieure (93) et un anneau intérieur (94) fixé à la paroi extérieure par des éléments de bande espacés (98), l'anneau intérieur comprenant des pattes/éléments flexibles (100) entre les éléments de bande qui s'étendent vers le bas et sont agencés et configurés pour permettre à une première partie effilée de la saillie de se déplacer entre celles-ci/ceux-ci en écartant les éléments de jambe vers l'extérieur, dans lequel les éléments de jambe reviennent en arrière contre la saillie, en maintenant l'ensemble d'embout de manière ferme sur la saillie.

14. Appareil selon la revendication 13, dans lequel les éléments de jambe sont équidistants et suffisamment flexibles pour permettre à l'ensemble formant embout d'être enlevé de et installé sur la saillie, tout en maintenant l'ensemble formant embout contre la saillie en fonctionnement, pour empêcher un mouvement de perte.
